# EUROPÄISCHE PATENTSCHRIFT

(11) **EP 1 773 213 B1**
(45) Veröffentlichungstag und Bekanntmachung des Hinweises auf die Patenterteilung: **02.05.2012**
(21) Anmeldenummer: 05763313.3
(22) Anmeldetag: 16.07.2005
(51) Int. Cl.: A61B 17/16, H02P 6/24

(54) **CHIRURGISCHE MASCHINE**
SURGICAL MACHINE
MACHINE CHIRURGICALE

(30) Priorität: 30.07.2004 DE 102004038414
(43) Veröffentlichungstag der Anmeldung: 18.04.2007
(73) Patentinhaber: Aesculap AG, 78532 Tuttlingen (DE)
(72) Erfinder: SCHNEIDER, Jürgen, 78532 Tuttlingen (DE); HÖGERLE, Roland, Alois, 78532 Tuttlingen (DE); KONRATH, Harald, 72108 Rottenburg-Hailfingen (DE)
(74) Vertreter: Hoeger, Stellrecht & Partner Patentanwälte
(86) Internationale Anmeldenummer: PCT/EP2005/007760
(87) Internationale Veröffentlichungsnummer: WO 2006/012991

(56) Entgegenhaltungen:
- WO-A-96/01521
- WO-A-97/50171
- WO-A-2004/036755
- US-A- 5 268 622
- US-A- 5 689 159
- US-A1- 2002 044 472

## Beschreibung

Die vorliegende Erfindung betrifft eine chirurgische Maschine mit einem Elektromotor, welcher einen Rotor und mindestens zwei Motorwicklungen aufweist, und mit einer Motorsteuerung zum Steuern und/oder Regeln des Elektromotors, wobei ein Gesamtdrehzahlbereich der chirurgischen Maschine in mindestens einen unteren Drehzahlbereich für niedrige Drehzahlen und in mindestens einen oberen Drehzahlbereich für höhere Drehzahlen als der mindestens eine untere Drehzahlbereich unterteilt ist, wobei die Motorsteuerung derart ausgebildet ist, daß in dem mindestens einen unteren Drehzahlbereich ein erstes Steuer- und/oder Regelungsverfahren zum Steuern und/oder Regeln des Elektromotors durchführbar ist.

Ferner betrifft die vorliegende Erfindung ein Verfahren zum Betreiben einer chirurgischen Maschine mit einem Elektromotor, welcher einen Rotor und mindestens zwei Motorwicklungen aufweist, und mit einer Motorsteuerung zum Steuern und/oder Regeln des Elektromotors, wobei ein Gesamtdrehzahlbereich der chirurgischen Maschine in mindestens einen unteren Drehzahlbereich für niedrige Drehzahlen und in mindestens einen oberen Drehzahlbereich für höhere Drehzahlen als im mindestens einen unteren Drehzahlbereich unterteilt wird, daß in dem mindestens einen unteren Drehzahlbereich ein erstes Steuer- und/oder Regelungsverfahren zum Steuern und/oder Regeln des Elektromotors durchgeführt wird.

Chirurgische Maschinen der eingangs beschriebenen Art sind in zahlreichen Varianten bekannt, insbesondere als Bohr- und Fräsmaschinen oder Sägen.

Sie werden betrieben, indem mit der Motorsteuerung Steuersignale für den Elektromotor erzeugt werden, um ihn mit einer bestimmten Drehzahl zu betreiben. Je nach Art des Elektromotors können Drehzahlen bis zu 70.000 Umdrehungen pro Minute erreicht werden. Konstruktionsbedingt ist jedoch der Wirkungsgrad von Elektromotoren nicht bei allen Drehzahlen gleich, insbesondere nicht immer optimal.

Eine chirurgische Maschine der eingangs beschriebenen Art ist beispielsweise aus der US 5,268,622 bekannt. In der WO 96/01521 A1 ist ein Verfahren zur Steuerung bürstenloser Gleichstrommotoren offenbart.

Es ist daher Aufgabe der vorliegenden Erfindung, eine chirurgische Maschine und ein Verfahren zum Betreiben einer chirurgischen Maschine so zu verbessern, daß insbesondere ein Wirkungsgrad des Elektromotors im wesentlichen über den gesamten Drehzahlbereich optimiert werden kann.

Diese Aufgabe wird bei einer chirurgischen Maschine der eingangs beschriebenen Art erfindungsgemäß dadurch gelöst, daß in dem mindestens einen oberen Drehzahlbereich ein zweites Steuer- und/oder Regelungsverfahren zum Steuern und/oder Regeln des Elektromotors durchführbar ist und daß das erste Steuer- und/oder Regelungsverfahren ein Space-Vektor-Puls-Weiten-Modulations-(SVPWM)-Verfahren ist, bei welchem alle Motorwicklungen gleichzeitig bestrombar sind.

Die erfindungsgemäße Weiterbildung bekannter chirurgischer Maschinen hat den Vorteil, daß jeweils an einen Drehzahlbereich des Elektromotors angepaßte Steuer- und/oder Regelungsverfahren angewandt werden können. Es wäre insbesondere denkbar, daß mehr als zwei Drehzahlbereiche definiert werden, wobei jeweils beim Übergang von einem zum anderen Drehzahlbereich auch das jeweils angewandte Steuer- und/oder Regelungsverfahren umgeschaltet wird. Auf diese Weise läßt sich nicht nur der Wirkungsgrad des Elektromotors im Betrieb optimieren, sondern es läßt beispielsweise auch eine Ist-Drehzahl des Elektromotors während des Betriebs in Abhängigkeit der Drehzahl optimiert bestimmen. Das SVPWM-Verfahren hat gegenüber herkömmlichen Puls-Weiten-Modulations-(PWM)-Verfahren den Vorteil, daß alle Motorwicklungen gleichzeitig bestrombar sind, so daß auch bei besonders niedrigen Drehzahlen ein sanfter, ruckelfreier Betrieb des Elektromotors möglich wird. Ferner wird ein Anlaufen des Motors aus dem Stillstand wesentlich dadurch verbessert, daß alle Motorwicklungen gleichzeitig bestrombar sind.

Vorteilhaft ist es, wenn das erste und/oder das zweite Steuer- und/oder Regelungsverfahren ein Puls-Weiten-Modelations-(PWM)-Verfahren ist. Mit diesem Verfahren lassen sich insbesondere Gleichstrommotoren auf einfache Weise und optimiert betreiben. Insbesondere lassen sich sinusartige Strom- und Spannungsverläufe erzeugen durch Überlagerung digitaler Spannungs- bzw. Stromsignale mit einer Trägerfrequenz.

Besonders kostengünstig und wenig wartungsintensiv wird die Maschine, wenn der Elektromotor ein bürstenloser Gleichstrommotor ist.

Vorzugsweise ist der Elektromotor ein sensorloser Elektromotor. Darunter ist zu verstehen, daß keine Drehzahlerfassungssensoren zur Bestimmung einer Ist-Drehzahl des Elektromotors vorgesehen oder am Elektromotor angeordnet sind. Derartige Elektromotoren sind wesentlich kostengünstiger als Motoren, die Sensoren umfassen, und zudem vereinfacht sich der Aufbau der chirurgischen Maschine insgesamt. Grund hierfür ist, daß weniger Anschlüsse für den Motor vorgesehen werden müssen. Dies hat zudem den Vorteil, daß bei einer zerlegbarer chirurgischen Maschine keine Korrosionsprobleme bei Kontakten auftreten können, über welche eine Verbindung der Motorsteuerung zu Drehzahlerfassungs- und/oder Positionssensoren hergestellt werden kann. Derartige Kontakte werden üblicherweise mit kleinen Spannungen beziehungsweise Strömen beaufschlagt, so daß schon eine geringe Korrosion der Kontakte zu Fehlern bei der Ermittlung der Ist-Drehzahl des Elektromotors führen kann. Genau dies kann bei einer erfindungsgemäßen Maschine nicht passieren.

Alternativ kann in vorteilhafter Weise vorgesehen sein, daß der Motor Drehzahlerfassungssensoren aufweist und daß die Motorsteuerung derart ausgebildet ist, daß das erste Steuer- und/oder Regelungsverfahren ein Verfahren zum Steuern und/oder Regeln der chirurgischen Maschine ist, bei welchem die Motorsteuerung Steuersignale für den Elektromotor in Abhängigkeit einer mit den Drehzahlerfassungssensoren ermittelten Ist-Drehzahl bereitstellt. Die Drehzahlerfassungssensoren können auch dazu dienen, eine Position des Rotors des Elektromotors zu bestimmen. Drehzahlerfassungssensoren insbesondere bei niedrigen Drehzahlen des Elektromotors einzusetzen, hat den Vorteil, daß die Drehzahl wesentlich genauer bestimmt werden kann als beispielsweise durch Ermittlung einer an der oder den Motorwicklungen erzeugten Gegen-EMK (Elektro-Motorische-Kraft). Die Ermittlung der Gegen-EMK ist insbesondere bei höheren Drehzahlen besser geeignet, da in diesem Fall höhere Induktionsspannungen erzeugt werden und sich die ermittelten Signale dadurch besser verarbeiten lassen.

Ein besonders einfacher Aufbau der Maschine ergibt sich, wenn ein Hall-System zur Erfassung einer Ist-Drehzahl des Elektromotors vorgesehen ist und wenn das Hall-System die Drehzahlerfassungssensoren umfaßt. Hall-Sensoren als Drehzahlerfassungssensoren können besonders klein ausgebildet und direkt in den Elektromotor integriert werden.

Gemäß einer bevorzugten Ausführungsform der Erfindung kann vorgesehen sein, daß ein Drehzahlgrenzwert zwischen dem mindestens einen unteren und dem mindestens einen oberen Drehzahlbereich unveränderbar ist. In diesem Fall kann ein Umschalten zwischen den mindestens zwei Steuer- und/oder Regelungsverfahren immer bei einem gewünschten Drehzahlgrenzwert stattfinden.

Gemäß einer weiteren bevorzugten Ausführungsform der Erfindung kann auch vorgesehen sein, daß ein Drehzahlgrenzwert zwischen dem mindestens einen unteren und dem mindestens einen oberen Drehzahlbereich veränderbar ist. Je nach Betriebssituation läßt sich dadurch ein Umschalten zwischen den mindestens zwei Steuer- und/oder Regelungsverfahren gezielt verändern. Schaltpunkte können dann in gewünschter Weise variiert werden.

Ein dauerndes Umschalten zwischen den mindestens zwei Steuer- und/oder Regelungsverfahren läßt sich auf einfach Weise dadurch vermeiden, daß die Motorsteuerung derart ausgebildet ist, daß eine Umschaltung vom ersten Steuer- und/oder Regelungsverfahren in das zweite Steuer- und/oder Regeiungsverfahren bei einer ersten Umschaltdrehzahl erfolgt und daß eine Umschaltung vom zweiten Steuer- und/oder Regelungsverfahren in das erste Steuer- und/oder Regelungsverfahren bei einer zweiten Umschaltdrehzahl erfolgt. Es lassen sich so zwei Schaltpunkte definieren, und zwar beim Übergang vom niedrigen Drehzahlbereich in den höheren Drehzahlbereich und umgekehrt. Es ist so möglich, die Umschaltzeitpunkte zu trennen, das heißt eine kleine Variation der Ist-Drehzahl des Motors führt nicht zwangsläufig sofort zum Umschalten in das andere Steuer- und/oder Regelungsverfahren. Grundsätzlich wäre es denkbar, daß die erste Umschaltdrehzahl kleiner als die zweite Umschaltdrehzahl ist. Besonders günstig ist es jedoch, wenn die erste Umschaltdrehzahl gleich oder größer als die zweite Umschaltdrehzahl ist. Ein Umschalten in den höheren Drehzahlbereich findet somit bei einer höheren Umschaltdrehzahl statt als das Umschalten vom höheren Drehzahlbereich in den niedrigeren Drehzahlbereich. Es ergibt sich somit quasi ein Hysteresekurve mit einem Bereich, in welchem für bestimmte Drehzahlen sowohl das eine als auch das andere Steuer- und/oder Regelungsverfahren angewandt wird, allerdings in Abhängigkeit davon, ob die Drehzahl des Elektromotors zu- oder abnimmt.

Prinzipiell wäre es möglich, die Maschine so auszubilden, daß eine Bedienperson einen gewünschten Drehzahlbereich vorgibt und das entsprechende Steuer- und/oder Regelungsverfahren aktiviert. Gemäß einer bevorzugten Ausführungsform der Erfindung kann jedoch vorgesehen sein, daß die Motorsteuerung derart ausgebildet ist, daß die Umschaltung vom ersten Steuer- und/oder Regelungsverfahren in das zweite Steuer- und/oder Regelungsverfahren beim Übergang vom mindestens einen unteren in den mindestens einen oberen Drehzahlbereich, und umgekehrt, automatisch erfolgt. Die Bedienperson muß bei dieser erfindungsgemäßen Ausgestaltung der Maschine lediglich die Drehzahl vorgeben, bei weicher sie die Maschine betreiben möchte.

Besonders einfach wird der Aufbau der Maschine, wenn der Elektromotor drei Motorwicklungen aufweist. Derartige Elektromotoren sind in zahlreichen Ausführungsvarianten, insbesondere auch kostengünstig, verfügbar.

Die eingangs gestellte Aufgabe wird bei einem Verfahren der eingangs beschriebenen Art erfindungsgemäß dadurch gelöst, daß in dem mindestens einen oberen Drehzahlbereich ein zweites Steuer- und/oder Regelungsverfahren zum Steuern und/oder Regeln des Elektromotors durchgeführt wird und daß das erste Steuer- und/oder Regelungsverfahren ein Space-Vektor-Puls-Weiten-Modulations-(SVPWM)-Verfahren ist, bei welchem alle Motorwicklungen gleichzeitig bestromt werden.

Mit dem erfindungsgemäßen Verfahren kann eine chirurgische Maschine vorteilhaft betrieben werden, insbesondere läßt sich so ihr Wirkungsgrad insgesamt steigern, denn in Abhängigkeit der Drehzahl des Elektromotors kann das jeweils am besten geeignete Steuer- und Regelungsverfahren ausgewählt werden. Insbesondere bei niedrigen Drehzahlen und beim Anlaufen des Elektromotors ist es besonders günstig, daß das erste Steuer- und/oder Regelungsverfahren ein Space-Vektor-Puls-Weiten-Modulations-(SVPWM)-Verfahren ist, bei welchem alle Motorwicklungen gleichzeitig bestromt werden. Dieses Verfahren gestattet es, insbesondere bei niedrigen Drehzahlen, einen verbesserten Wirkungsgrad des Motors zu erreichen, da in Abhängigkeit der Rotorposition im Vergleich zu herkömmlich bestromten Elektromotoren auf den Rotor von den bestromten Motorwicklungen ausgeübte Kräfte optimiert werden können.

Auf besonders einfache Weise läßt sich ein Gleichstrommotor ansteuern, wenn das erste und/oder das zweite Steuer- und/oder Regelungsverfahren Puls-Weiten-Modulations-(PWM)-Verfahren sind.

Vorzugsweise wird ein Elektromotor verwendet, der ein bürstenloser Gleichstrommotor ist. Derartige Motoren sind kostengünstig und wartungsfreundlich.

Die Zahl von Kontakten an der chirurgischen Maschine läßt sich verringern, wenn ein Elektromotor verwendet wird, der ein sensorloser Elektromotor ist. Ferner sind derartige Motoren wesentlich kostengünstiger in der Herstellung.

Gemäß einer weiteren bevorzugten Variante des Verfahrens kann vorgesehen sein, daß der Motor Drehzahlerfassungssensoren aufweist und daß das erste Steuer- und/oder Regelungsverfahren ein Verfahren zum Steuern und/oder Regeln der chirurgischen Maschine ist, bei welchem die Motorsteuerung Steuersignale für den Elektromotor in Abhängigkeit einer mit den Drehzahlerfassungssensoren ermittelten Ist-Drehzahl bereitstellt. Insbesondere bei niedrigen Drehzahlen läßt sich mit Drehzahlerfassungssensoren eine Ist-Drehzahl des Elektromotors einfach und genau bestimmten.

Besonders einfach wird die Durchführung des Verfahrens, wenn ein Hall-System zur Erfassung einer Ist-Drehzahl des Elektromotors vorgesehen ist und wenn das Hall-System die Drehzahlerfassungssensoren umfaßt. Hall-Systeme haben sich in der Praxis in zahlreichen Anwendungen bewährt. Insbesondere können Hall-Sensoren besonders klein ausgebildet und direkt in den Elektromotor integriert werden.

Vorzugsweise bleibt ein Drehzahlgrenzwert zwischen dem mindestens einen unteren und dem mindestens einen oberen Drehzahlbereich beim Betrieb der Maschine unverändert. Das Verfahren wird auf diese Weise maximal vereinfacht.

Gemäß einer weiteren bevorzugten Variante des erfindungsgemäßen Verfahrens kann jedoch vorgesehen sein, daß ein Drehzahlgrenzwert zwischen dem mindestens einen unteren und dem mindestens einen oberen Drehzahlbereich im Betrieb der Maschine verändert wird. Dieses Verfahren ermöglicht es, einen Umschaltdrehzahlwert entsprechend einer aktuellen Betriebssituation festzusetzen.

Günstig ist es, wenn eine Umschaltung vom ersten Steuer- und/oder Regelungsverfahren in das zweite Steuer- und/oder Regelungsverfahren bei einer ersten Umschaltdrehzahl erfolgt und wenn eine Umschaltung vom zweiten Steuer- und/oder Regelungsverfahren in das erste Steuer- und/oder Regelungsverfahren bei einer zweiten Umschaltdrehzahl erfolgt. Dadurch lassen sich Umschaltpunkte beim Übergang vom unteren in den oberen Drehzahlbereich, und umgekehrt, in gewünschter Weise einstellen. Insbesondere kann so ein häufiges Umschalten vermieden werden, wenn die Maschine mit Drehzahlen im Bereich der Umschaltdrehzahl betrieben wird. Ein ständiges Umschalten würde den Betrieb der chirurgischen Maschine negativ beeinflussen, insbesondere eine Laufruhe stören.

Vorzugsweise ist die erste Umschaltdrehzahl gleich oder größer als die zweite Umschaltdrehzahl. Damit erfolgt eine Umschaltung vom niedrigen Drehzahlbereich in den hohen Drehzahlbereich vorzugsweise bei einer höheren Drehzahl als umgekehrt.

Vorteilhaft ist es, wenn die Umschaltung vom ersten Steuer- und/oder Regelungsverfahren in das zweite Steuer- und/oder Regelungsverfahren beim Übergang vom mindestens einen unteren in den mindestens einen oberen Drehzahlbereich, oder umgekehrt, automatisch erfolgt. Eine Bedienperson muß in diesem Fall lediglich eine gewünschte Drehzahl für die chirurgische Maschine vorgeben, sich jedoch nicht um eine mögliche Umschaltung zwischen unterschiedlichen Steuer- und/oder Regelungsverfahren krümmern.

Besonders einfach wird die Durchführung des Verfahrens, wenn ein Elektromotor mit drei Motorwicklungen verwendet wird.

Die nachfolgende Beschreibung einer bevorzugten Ausführungsform der Erfindung dient im Zusammenhang mit der Zeichnung der näheren Erläuterung. Es zeigen:
- Figur 1:: Eine schematische Darstellung einer chirurgischen Akkumaschine;
- Figur 2:: ein Schaltdiagramm einer Motorsteuerung der in Figur 1 dargestellten Akkumaschine; und
- Figur 3:: ein zum Schaltdiagramm in Figur 2 korrespondierender Ablaufplan für einen Betrieb der in Figur 1 dargestellten Akkumaschine.

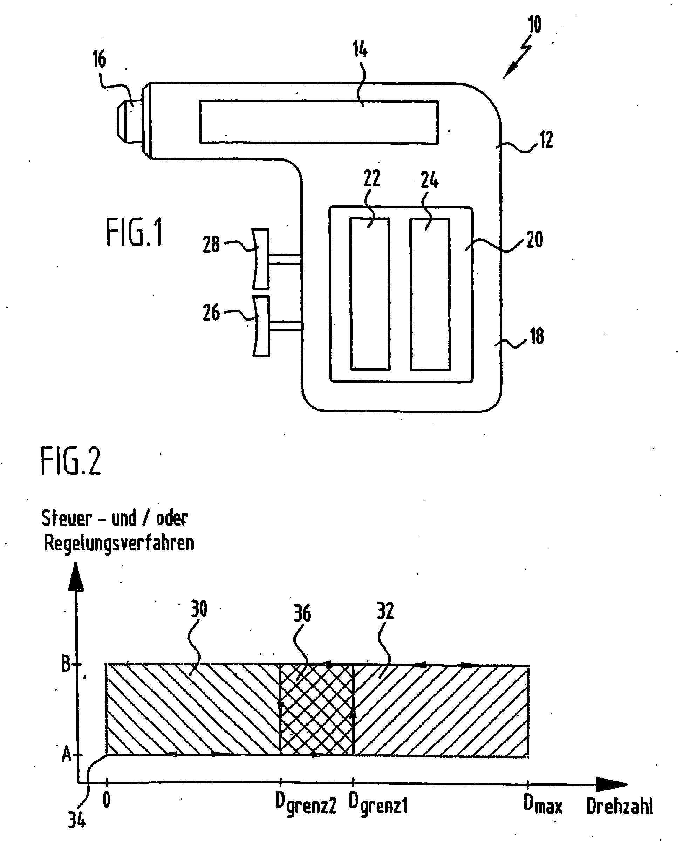

In Figur 1 ist eine insgesamt mit dem Bezugszeichen 10 versehene chirurgische Akkumaschine dargestellt, die ein Gehäuse 12 aufweist, in dessen einem Teil ein Elektromotor 14 parallel zur Längsachse dieses Gehäuseteils angeordnet ist, welcher eine nicht dargestellte Antriebswelle der Akkumaschine 10 antreibt. Am Ende der Antriebswelle ist eine Kupplung 16 angeordnet, mittels welcher die Akkumaschine 10 mit Werkzeugen jedweder Art verbunden werden kann, beispielsweise Bohrern, Fräsern, Meißeln und, unter Umständen über spezielle Kupplungen, auch mit Sägeblättern.

Von dem den Elektromotor 14 aufnehmenden Gehäuseteil des Gehäuses 12 steht quer ein Handgriff 18 ab, in welchen ein Powerpack 20 einführbar ist. Der Powerpack 20 umfaßt eine wiederaufladbare Batterie 22 sowie eine Motorsteuerung 24. Zur Inbetriebnahme der Akkumaschine 10 sind ein Gasdrücker 26 und ein Betriebsmodiwahlschalter 28 vorgesehen, welche im wesentlichen parallel zu einer Längsachse des Elektromotors 14 in den Handgriff 18 hineingedrückt werden können.

Bei dem Elektromotor 14 handelt es sich um einen sensorlosen Motor, das heißt es sind keine Drehzahlerfassungssensoren zur Detektion einer Rotorbewegung und einer Position eines Rotors des Elektromotors 14 vorgesehen.

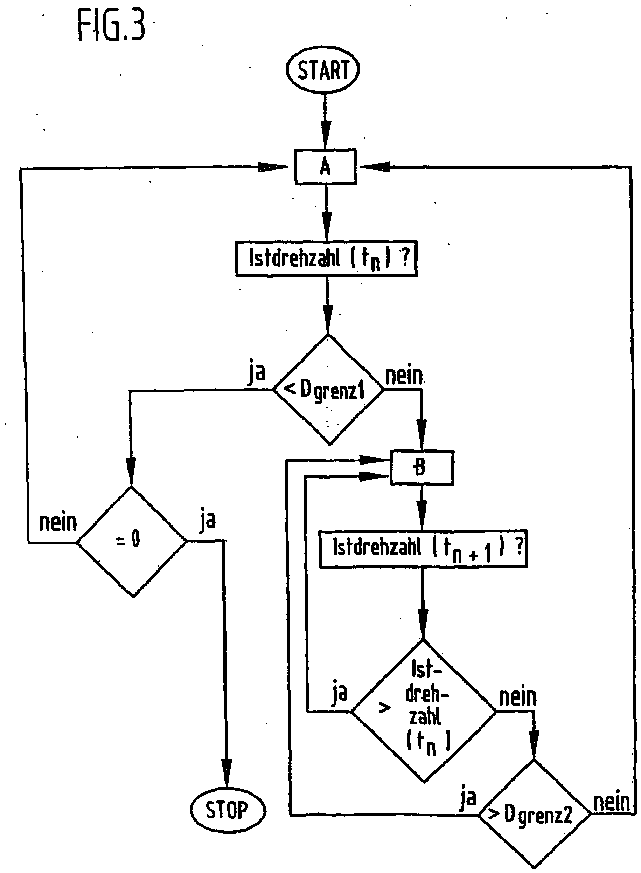

Die Motorsteuerung 24 ist derart ausgebildet, daß ein Drehzahlbereich des Elektromotors 14 in zwei Teilbereiche unterteilt wird, nämlich einen unteren Drehzahlbereich 30 und einen oberen Drehzahlbereich 32, wie dies in Figur 2 schematisch dargestellt ist. Des weiteren gestattet es die Motorsteuerung 24, zwei unterschiedliche Steuer- und/oder Regelungsverfahren zum Betreiben des Elektromotors 14 auszuführen. Dies ist zum einen ein Space-Vektor-Puls-Weiten-Modulations-(SVPWM)-Verfahren, welches in den Figuren 2 und 3 schematisch mit A bezeichnet ist. Zum anderen handelt es sich um ein herkömmliches Puls-Weiten-Moluations-(PWM)-Verfahren, welches in den Figuren 2 und 3 schematisch mit B gekennzeichnet ist.

Im Falle eines Elektromotors 14 mit einem Drehzahlerfassungssystem, welches Positionssensoren und Drehzahlerfassungssensoren aufweist, könnte das Steuer- und/oder Regelungsverfahren A auch ein Steuer- und/oder Regelungsverfahren sein, bei welchem eine Ist-Drehzahl des Elektromotors 14 mittels der Drehzahlerfassungssensoren ermittelt und von der Motorsteuerung 24 verarbeitet wird. Beim Space-Vektor-Puls-Weiten-Molulations-(SVPWM)-Verfahren und auch beim herkömmlichen Puls-Weiten-Modulations-(PWM)-Verfahren wird eine Ist-Drehzahl des Elektromotors 14 durch Bestimmung der Gegen-EMK ermittelt.

Mit Bezug zu den Figuren 2 und 3 wird nachfolgend die Vorgehensweise beim Umschalten vom Steuer- und/oder Regelungsverfahren A auf das Steuer- und/oder Regelungsverfahren B näher erläutert.

Betätigen des Gasdrückers 26 durch eine Bedienperson setzt die Akkumaschine 10 in Betrieb. In Figur 2 sind Start/Stop mit dem Bezugszeichen 34 versehen. Erhöht die Bedienperson die Drehzahl des Elektromotors 14, so führt die Motorsteuerung 24 das Steuer- und/oder Regelungsverfahren A aus bis zum Erreichen der Umschaltdrehzahl D_{grenz1}. Sobald die Umschaltdrehzahl D_{grenz1} erreicht ist, schaltet die Motorsteuerung 24 automatisch auf das Steuer- und/oder Regelungsverfahren B um. Bis zum Erreichen der Maximaldrehzahl Dₘₐₓ des Elektromotors 14 wird der Elektromotor 14 von der Motorsteuerung 24 im Steuer- und/oder Regelungsverfahren B betrieben. Wird die Drehzahlanforderung für den Elektromotor 14 durch die Bedienperson wieder herabgesetzt, so wird auch für Drehzahlen des Elektromotors 14, die kleiner sind als die Umschaltdrehzahl D_{grenz1} das Steuer- und/oder Regelungsverfahren B beibehalten, bis die Umschaltdrehzahl D_{grenz2} erreicht wird. Erst bei Erreichen der Umschaltdrehzahl D_{grenz2} und Unterschreiten derselben schaltet die Motorsteuerung 24 wieder auf das Steuer- und/oder Regelungsverfahren A um. Wird die Drehzahlanforderung wieder erhöht, dann erfolgt jedoch eine Umschaltung auf das Steuer- und/oder Regelungsverfahren B erst wieder nach Überschreiten der Umschaltdrehzahl D_{grenz1}.

Folge dieses Schaltschemas ist, daß in Figur 1 ein Überlappbereich zwischen dem unteren Drehzahlbereich 30 und dem oberen Drehzahlbereich 32 gebildet wird, der insgesamt mit dem Bezugszeichen 36 versehen ist. Im Überlappbereich 36 kann die Motorsteuerung 24 sowohl das Steuer- und/oder Regelungsverfahren A als auch das Steuer- und/oder Regelungsverfahren B ausführen. Welches Verfahren ausgeführt wird, hängt jedoch davon ab, ob die Drehzahlanforderung ausgehend von einer Ist-Drehzahl unterhalb der Umschaltdrehzahl D_{grenz2} erhöht oder von oberhalb der Umschaltdrehzahl D_{grenz1} abgesenkt wird. Insgesamt ergibt sich die in Figur 2 dargestellte hystereseartige Kurve, auf welcher der Überlappbereich 36 im Gegenuhrzeigersinn umwandert werden kann.

Die Funktionsweise der Motorsteuerung 24 zum Umschalten zwischen den beiden Steuer- und/oder Regelungsverfahren A und B wird anhand Figur 3 deutlich. Ausgangspunkt ist ein stillstehender Elektromotor 14. Wird dieser gestartet, so führt die Motorsteuerung 24 das Steuer- und/oder Regelungsverfahren A aus. Die Ist-Drehzahl zum Zeitpunkt tₙ wird in periodischen Abständen ermittelt. Nach Ermittlung der Ist-Drehzahl zum Zeitpunkt tₙ wird abgefragt, ob die Ist-Drehzahl kleiner als die Umschaltdrehzahl D_{grenz1}. Ist die Drehzahl kleiner als die Umschaltdrehzahl D_{grenz1}, dann wird abgefragt, ob die Drehzahl gleich 0 ist. Ist dies der Fall, dann stoppt die Motorsteuerung 24 den Betrieb des Elektromotors 14. Ist die Ist-Drehzahl kleiner als die Umschaltdrehzahl D_{grenz1}, jedoch größer als 0, dann wird weiter das Steuer- und/oder Regelungsverfahren A ausgeführt.

Ist die ermittelte Ist-Drehzahl zum Zeitpunkt tₙ größer als die Umschaltdrehzahl D_{grenz1}, dann schaltet die Motorsteuerung 24 auf das Steuer- und/oder Regelungsverfahren B um. Die Ist-Drehzahl zum Zeitpunkt tₙ₊₁ wird weiterhin in periodischen Abständen bestimmt und anschließend mit der zuvor bestimmten Ist-Drehzahl zum Zeitpunkt tₙ verglichen. Ist die Ist-Drehzahl zum Zeitpunkt tₙ₊₁ größer als die Ist-Drehzahl zum Zeitpunkt tₙ, dann führt die Motorsteuerung 24 weiterhin das Steuer- und/oder Regelungsverfahren B aus. Ist die Ist-Drehzahl zum Zeitpunkt tₙ₊₁ jedoch kleiner als die Ist-Drehzahl zum Zeitpunkt tₙ, dann wird die Ist-Drehzahl mit der Umschaltdrehzahl D_{grenz2} verglichen. Ist die Ist-Drehzahl größer als die Umschaltdrehzahl D_{grenz2} dann führt die Motorsteuerung weiterhin das Steuer- und/oder Regelungsverfahren B aus. Andernfalls schaltet die Motorsteuerung 24 automatisch auf das Steuer- und/oder Regelungsverfahren A um.

Das Umschalten zwischen den beiden Steuer- und/oder Regelungsverfahren A und B hat insbesondere den Vorteil, daß ein bei niedrigen Drehzahlen durchgeführtes Space-Vektor-Puls-Weiten-Modulations-(SVPWM)-Verfahren bei hohen Drehzahlen unerwünschte Dämpfungseffekte zeigt, wodurch sich Motorverluste ergeben und der Wirkungsgrad der Akkumaschine (10) nachteilig beeinflußt wird.

Die beiden Steuer- und/oder Regelungsverfahren A und B können in der Motorsteuerung hardware- oder softwaremäßig implementiert sein.

## Patentansprüche

1. Chirurgische Maschine (10) mit einem Elektromotor (14), welcher einen Rotor und mindestens zwei Motorwicklungen aufweist, und mit einer Motorsteuerung (24) zum Steuern und/oder Regeln des Elektromotors (14), wobei ein Gesamtdrehzahlbereich (30, 32) der chirurgischen Maschine (10) in mindestens einen unteren Drehzahlbereich (30) für niedrige Drehzahlen und in mindestens einen oberen Drehzahlbereich (32) für höhere Drehzahlen als der mindestens eine untere Drehzahlbereich (30) unterteilt ist, wobei die Motorsteuerung (24) derart ausgebildet ist, daß in dem mindestens einen unteren Drehzahlbereich (30) ein erstes Steuer- und/oder Regelungsverfahren (A) zum Steuern und/oder Regeln des Elektromotors (14) durchführbar ist, **dadurch gekennzeichnet, daß** in dem mindestens einen oberen Drehzahlbereich (32) ein zweites Steuer- und/oder Regelungsverfahren (B) zum Steuern und/oder Regeln des Elektromotors (14) durchführbar ist und daß das erste Steuer- und/oder Regelungsverfahren (A) ein Space-Vektor-Puls-Weiten-Modulations-(SVPWM)-Verfahren ist, bei welchem alle Motorwicklungen gleichzeitig bestrombar sind.

2. Maschine nach Anspruch 1, **dadurch gekennzeichnet, daß** das erste und/oder das zweite Steuer- und/oder Regelungsverfahren (A, B) ein Puls-Weiten-Modulations-(PWM)-Verfahren ist.

3. Maschine nach einem der voranstehenden Ansprüche, **dadurch gekennzeichnet, daß** der Elektromotor (14) ein bürstenloser Gleichstrommotor ist.

4. Maschine nach einem der voranstehenden Ansprüche, **dadurch gekennzeichnet, daß** der Elektromotor (14) ein sensorloser Elektromotor ist.

5. Maschine nach einem der Ansprüche 1 bis 3, **dadurch gekennzeichnet, daß** der Elektromotor (14) Drehzahlerfassungssensoren aufweist und daß die Motorsteuerung (24) derart ausgebildet ist, daß das erste Steuer- und/oder Regelungsverfahren (A) ein Verfahren zum Steuern und/oder Regeln der chirurgischen Maschine (10) ist, bei welchem die Motorsteuerung (24) Steuersignale für den Elektromotor (14) in Abhängigkeit einer mit den Drehzahlerfassungssensoren ermittelten Ist-Drehzahl bereitstellt.

6. Maschine nach Anspruch 5, **dadurch gekennzeichnet, daß** ein Hall-System zur Erfassung einer Ist-Drehzahl des Elektromotors (14) vorgesehen ist und daß das Hall-System die Drehzahlerfassungssensoren umfaßt.

7. Maschine nach einem der voranstehenden Ansprüche, **dadurch gekennzeichnet, daß** ein Drehzahlgrenzwert (D_{grenz1}, D_{grenz2}) zwischen dem mindestens einen unteren und dem mindestens einen oberen Drehzahlbereich (30, 32) unveränderbar ist.

8. Maschine nach einem der Ansprüche 1 bis 6, **dadurch gekennzeichnet, daß** ein Drehzahlgrenzwert (D_{grenz1}, D_{grenz2}) zwischen dem mindestens einen unteren und dem mindestens einen oberen Drehzahlbereich (30, 32) veränderbar ist.

9. Maschine nach einem der voranstehenden Ansprüche, **dadurch gekennzeichnet, daß** die Motorsteuerung (24) derart ausgebildet ist, daß eine Umschaltung vom ersten Steuer- und/oder Regelungsverfahren (A) in das zweite Steuer- und/oder Regelungsverfahren (B) bei einer ersten Umschaltdrehzahl (D_{grenz1}) erfolgt und daß eine Umschaltung vom zweiten Steuer- und/oder Regelungsverfahren (B) in das erste Steuer- und/oder Regelungsverfahren (A) bei einer zweiten Umschaltdrehzahl (D_{grenz2}) erfolgt.

10. Maschine nach Anspruch 9, **dadurch gekennzeichnet, daß** die erste Umschaltdrehzahl (D_{grenz1}) gleich oder größer als die zweite Umschaltdrehzahl (D_{grenz2}) ist.

11. Maschine nach einem der voranstehenden Ansprüche, **dadurch gekennzeichnet, daß** die Motorsteuerung (24) derart ausgebildet ist, daß die Umschaltung vom ersten Steuer- und/oder Regelungsverfahren (A) in das zweite Steuer- und/oder Regelungsverfahren (B) beim Übergang vom mindestens einen unteren in den mindestens einen oberen Drehzahlbereich (30, 32) und umgekehrt, automatisch erfolgt.

12. Maschine nach einem der voranstehenden Ansprüche, **dadurch gekennzeichnet, daß** der Elektromotor (14) drei Motorwicklungen aufweist.

13. Verfahren zum Betreiben einer chirurgischen Maschine mit einem Elektromotor, welcher einen Rotor und mindestens zwei Motorwicklungen aufweist, und mit einer Motorsteuerung zum Steuern und/oder Regeln des Elektromotors, wobei ein Gesamtdrehzahlbereich der chirurgischen Maschine in mindestens einen unteren Drehzahlbereich für niedrige Drehzahlen und in mindestens einen oberen Drehzahlbereich für höhere Drehzahlen als im mindestens einen unteren Drehzahlbereich unterteilt wird, wobei in dem mindestens einen unteren Drehzahlbereich ein erstes Steuer- und/oder Regelungsverfahren zum Steuern und/oder Regeln des Elektromotors durchgeführt wird, **dadurch gekennzeichnet, daß** in dem mindestens einen oberen Drehzahlbereich ein zweites Steuer- und/oder Regelungsverfahren zum Steuern und/oder Regeln des Elektromotors durchgeführt wird und daß das erste Steuer- und/oder Regelungsverfahren ein Space-Vektor-Puls-Weiten-Modulations-(SVPWM)-Verfahren ist, bei welchem alle Motorwicklungen gleichzeitig bestromt werden.

14. Verfahren nach Anspruch 13, **dadurch gekennzeichnet, daß** das erste und/oder das zweite Steuer- und/oder Regelungsverfahren Puls-Weiten-Modulations-(PWM)-Verfahren sind.

15. Verfahren nach Anspruch 13 oder 14, **dadurch gekennzeichnet, daß** ein Elektromotor verwendet wird, der ein bürstenloser Gleichstrommotor ist.

16. Verfahren nach einem der Ansprüche 13 bis 15, **dadurch gekennzeichnet, daß** ein Elektromotor verwendet wird, der ein sensorloser Elektromotor ist.

17. Verfahren nach einem der Ansprüche 13 bis 16, **dadurch gekennzeichnet, daß** der Motor Drehzahlerfassungssensoren aufweist und daß das erste Steuer- und/oder Regelungsverfahren ein Verfahren zum Steuern und/oder Regeln der chirurgischen Maschine ist, bei welchem die Motorsteuerung Steuersignale für den Elektromotor in Abhängigkeit einer mit den Drehzahlerfassungssensoren ermittelten Ist-Drehzahl bereitstellt.

18. Verfahren nach Anspruch 17, **dadurch gekennzeichnet, daß** ein Hall-System zur Erfassung einer Ist-Drehzahl des Elektromotors vorgesehen ist und daß das Hall-System die Drehzahlerfassungssensoren umfaßt.

19. Verfahren nach einem der Ansprüche 13 bis 18, **dadurch gekennzeichnet, daß** ein Drehzahlgrenzwert zwischen dem mindestens einen unteren und dem mindestens einen oberen Drehzahlbereich beim Betrieb der Maschine unverändert bleibt.

20. Verfahren nach einem der Ansprüche 13 bis 18, **dadurch gekennzeichnet, daß** ein Drehzahlgrenzwert zwischen dem mindestens einen unteren und dem mindestens einen oberen Drehzahlbereich im Betrieb der Maschine verändert wird.

21. Verfahren nach einem der Ansprüche 13 bis 20, **dadurch gekennzeichnet, daß** eine Umschaltung vom ersten Steuer- und/oder Regelungsverfahren in das zweite Steuer- und/oder Regelungsverfahren bei einer ersten Umschaltdrehzahl erfolgt und daß eine Umschaltung vom zweiten Steuer- und/oder Regelungsverfahren in das erste Steuer- und/oder Regelungsverfahren bei einer zweiten Umschaltdrehzahl erfolgt.

22. Verfahren nach Anspruch 21, **dadurch gekennzeichnet, daß** die erste Umschaltdrehzahl gleich oder größer als die zweite Umschaltdrehzahl ist.

23. Verfahren nach einem der Ansprüche 13 bis 22, **dadurch gekennzeichnet, daß** die Umschaltung vom ersten Steuer- und/oder Regelungsverfahren in das zweite Steuer- und/oder Regelungsverfahren beim Übergang vom mindestens einen unteren in den mindestens einen oberen Drehzahlbereich, und umgekehrt, automatisch erfolgt.

24. Verfahren nach einem der Ansprüche 13 bis 23, **dadurch gekennzeichnet, daß** ein Elektromotor mit drei Motorwicklungen verwendet wird.

## Claims

1. Surgical machine (10) with an electric motor (14) comprising a rotor and at least two motor windings, and with a motor controller (24) for controlling and/or regulating the electric motor (14), wherein an entire rotational speed range (30, 32) of the surgical machine (10) is divided into at least one lower rotational speed range (30) for low rotational speeds and at least one upper rotational speed range (32) for higher rotational speeds than those in the at least one lower rotational speed range (30), wherein the motor controller (24) is so designed that a first controlling and/or regulating method (A) for controlling and/or regulating the electric motor (14) is performable in the at least one lower rotational speed range (30), **characterized in that** a second controlling and/or regulating method (B) for controlling and/or regulating the electric motor (14) is performable in the at least one upper rotational speed range (32), and **in that** the first controlling and/or regulating method (A) is a space vector pulse width modulation (SVPWM) method in which all motor windings are able to be simultaneously supplied with electric current.

2. Machine in accordance with claim 1, **characterized in that** the first and/or the second controlling and/or regulating method (A, B) is a pulse width modulation (PWM) method.

3. Machine in accordance with any one of the preceding claims, **characterized in that** the electric motor (14) is a brushless DC motor.

4. Machine in accordance with any one of the preceding claims, **characterized in that** the electric motor (14) is a sensorless electric motor.

5. Machine in accordance with any one of claims 1 to 3, **characterized in that** the electric motor (14) comprises rotational speed detection sensors, and **in that** the motor controller (24) is so designed that the first controlling and/or regulating method (A) is a method for controlling and/or regulating the surgical machine (10), in which the motor controller (24) provides control signals for the electric motor (14) in dependence upon an actual rotational speed determined with the rotational speed detection sensors.

6. Machine in accordance with claim 5, **characterized in that** a Hall system is provided for detecting an actual rotational speed of the electric motor (14), and **in that** the Hall system comprises the rotational speed detection sensors.

7. Machine in accordance with any one of the preceding claims, **characterized in that** a rotational speed limit value (Dₗᵢₘᵢₜ₁, Dₗᵢₘᵢₜ₂) between the at least one lower rotational speed range (30) and the at least one upper rotational speed range (32) is unalterable.

8. Machine in accordance with any one of claims 1 to 6, **characterized in that** a rotational speed limit value (Dₗᵢₘᵢₜ₁, Dₗᵢₘᵢₜ₂) between the at least one lower rotational speed range (30) and the at least one upper rotational speed range (32) is alterable.

9. Machine in accordance with any one of the preceding claims, **characterized in that** the motor controller (24) is so designed that a switchover from the first controlling and/or regulating method (A) to the second controlling and/or regulating method (B) takes place at a first switchover rotational speed (Dₗᵢₘᵢₜ₁), and that a switchover from the second controlling and/or regulating method (B) to the first controlling and/or regulating method (A) takes place at a second switchover rotational speed (Dₗᵢₘᵢₜ₂).

10. Machine in accordance with claim 9, **characterized in that** the first switchover rotational speed (Dₗᵢₘᵢₜ₁) is equal to or greater than the second switchover rotational speed (Dₗᵢₘᵢₜ₂).

11. Machine in accordance with any one of the preceding claims, **characterized in that** the motor controller (24) is so designed that the switchover from the first controlling and/or regulating method (A) to the second controlling and/or regulating method (B) takes place automatically at the transition from the at least one lower rotational speed range (30) to the at least one upper rotational speed range (32), and vice versa.

12. Machine in accordance with any one of the preceding claims, **characterized in that** the electric motor (14) comprises three motor windings.

13. Method for operating a surgical machine with an electric motor comprising a rotor and at least two motor windings, and with a motor controller for controlling and/or regulating the electric motor, wherein an entire rotational speed range of the surgical machine is divided into at least one lower rotational speed range for low rotational speeds and at least one upper rotational speed range for higher rotational speeds than those in the at least one lower rotational speed range, wherein a first controlling and/or regulating method for controlling and/or regulating the electric motor is performed in the at least one lower rotational speed range, **characterized in that** a second controlling and/or regulating method for controlling and/or regulating the electric motor is performed in the at least one upper rotational speed range, and **in that** the first controlling and/or regulating method is a space vector pulse width modulation (SVPWM) method, in which all motor windings are simultaneously supplied with electric current.

14. Method in accordance with claim 13, **characterized in that** the first and/or the second controlling and/or regulating methods are pulse width modulation (PWM) methods.

15. Method in accordance with claim 13 or 14, **characterized in that** an electric motor, which is a brushless DC motor, is used.

16. Method in accordance with any one of claims 13 to 15, **characterized in that** an electric motor, which is a sensorless electric motor, is used.

17. Method in accordance with any one of claims 13 to 16, **characterized in that** the motor comprises rotational speed detection sensors, and **in that** the first controlling and/or regulating method is a method for controlling and/or regulating the surgical machine, in which the motor controller provides control signals for the electric motor in dependence upon an actual rotational speed determined with the rotational speed detection sensors.

18. Method in accordance with claim 17, **characterized in that** a Hall system is provided for detecting an actual rotational speed of the electric motor, and **in that** the Hall system comprises the rotational speed detection sensors.

19. Method in accordance with any one of claims 13 to 18, **characterized in that** a rotational speed limit value between the at least one lower rotational speed range and the at least one upper rotational speed range remains unaltered during operation of the machine.

20. Method in accordance with any one of claims 13 to 18, **characterized in that** a rotational speed limit value between the at least one lower rotational speed range and the at least one upper rotational speed range is altered during operation of the machine.

21. Method in accordance with any one of claims 13 to 20, **characterized in that** a switchover from the first controlling and/or regulating method to the second controlling and/or regulating method takes place at a first switchover rotational speed, and **in that** a switchover from the second controlling and/or regulating method to the first controlling and/or regulating method takes place at a second switchover rotational speed.

22. Method in accordance with claim 21, **characterized in that** the first switchover rotational speed is equal to or greater than the second switchover rotational speed.

23. Method in accordance with any one of claims 13 to 22, **characterized in that** the switchover from the first controlling and/or regulating method to the second controlling and/or regulating method takes place automatically at the transition from the at least one lower rotational speed range to the at least one upper rotational speed range, and vice versa.

24. Method in accordance with any one of claims 13 to 23, **characterized in that** an electric motor with three motor windings is used.

## Revendications

1. Machine chirurgicale (10) comprenant un moteur électrique (14), qui présente un rotor et au moins deux enroulements de moteur, et comprenant également une commande de moteur (24) destinée à commander et/ou réguler le moteur électrique (14), machine dans laquelle une plage globale de vitesse de rotation (30, 32) de la machine chirurgicale (10) est subdivisée en au moins une plage de vitesse de rotation inférieure (30) pour les faibles vitesses de rotation et au moins une plage de vitesse de rotation supérieure (32) pour des vitesses de rotation plus élevées que celles de ladite au moins une plage de vitesse de rotation inférieure (30), et dans laquelle la commande de moteur (24) est conçue de manière à pouvoir exécuter dans ladite au moins une plage de vitesse de rotation inférieure (30), un premier procédé de commande et/ou de régulation (A) pour commander et/ou réguler le moteur électrique (14), **caractérisée en ce que** dans ladite au moins une plage de vitesse de rotation supérieure (32) il est possible d'exécuter un deuxième procédé de commande et/ou de régulation (B) pour commander et/ou réguler le moteur électrique (14), et **en ce que** le premier procédé de commande et/ou de régulation (A) et un procédé de modulation de largeur d'impulsion selon le principe du vecteur spatial (procédé SVPWM), pour lequel tous les enroulements de moteur peuvent être alimentés en courant simultanément.

2. Machine selon la revendication 1, **caractérisée en ce que** le premier et/ou le deuxième procédé de commande et/ou de régulation (A, B) est un procédé de modulation de largeur d'impulsion (PWM).

3. Machine selon l'une des revendications précédentes, **caractérisée en ce que** le moteur électrique (14) est un moteur à courant continu sans balais.

4. Machine selon l'une des revendications précédentes, **caractérisée en ce que** le moteur électrique (14) est un moteur électrique sans détecteurs.

5. Machine selon l'une des revendications 1 à 3, **caractérisée en ce que** le moteur électrique (14) comporte des détecteurs de relevé de vitesse de rotation, et **en ce que** la commande de moteur (24) est conçue de manière à ce que le premier procédé de commande et/ou de régulation (A) soit un procédé pour commander et/ou réguler la machine chirurgicale (10) pour lequel la commande de moteur (24) fournit des signaux de commande pour le moteur électrique (14) en fonction d'une vitesse de rotation instantanée déterminée par les détecteurs de relevé de vitesse de rotation.

6. Machine selon la revendication 5, **caractérisée en ce qu'**il est prévu un système de Hall pour relever une vitesse de rotation instantanée du moteur électrique (14), et **en ce que** le système de Hall comprend les détecteurs de relevé de vitesse de rotation.

7. Machine selon l'une des revendications précédentes, **caractérisée en ce qu'**une valeur limite de vitesse de rotation (D_{grenz1}, D_{grenz2}) entre ladite au moins une plage de vitesse de rotation inférieure et ladite au moins une plage de vitesse de rotation supérieure (30, 32), ne peut être modifiée.

8. Machine selon l'une des revendications 1 à 6, **caractérisée en ce qu'**une valeur limite de vitesse de rotation (D_{grenz1}, D_{grenz2}) entre ladite au moins une plage de vitesse de rotation inférieure et ladite au moins une plage de vitesse de rotation supérieure (30, 32), peut être modifiée.

9. Machine selon l'une des revendications précédentes, **caractérisée en ce que** la commande de moteur (24) est conçue de manière à ce qu'une commutation du premier procédé de commande et/ou de régulation (A) au deuxième procédé de commande et/ou de régulation (B) s'effectue pour une première vitesse de rotation de commutation (D_{grenz1}), et qu'une commutation du deuxième procédé de commande et/ou de régulation (B) au premier procédé de commande et/ou de régulation (A) s'effectue pour une deuxième vitesse de rotation de commutation (D_{grenz2}).

10. Machine selon la revendication 9, **caractérisée en ce que** la première vitesse de rotation de commutation (D_{grenz1}) est égale ou supérieure à la deuxième vitesse de rotation de commutation (D_{grenz2}).

11. Machine selon l'une des revendications précédentes, **caractérisée en ce que** la commande de moteur (24) est conçue de manière à ce que la commutation du premier procédé de commande et/ou de régulation (A) au deuxième procédé de commande et/ou de régulation (B) s'effectue automatiquement lors du passage de ladite au moins une plage de vitesse de rotation inférieure à ladite au moins une plage de vitesse de rotation supérieure (30, 32) et inversement.

12. Machine selon l'une des revendications précédentes, **caractérisée en ce que** le moteur électrique (14) présente trois enroulements de moteur.

13. Procédé pour faire fonctionner une machine chirurgicale comprenant un moteur électrique, qui présente un rotor et au moins deux enroulements de moteur, et comprenant également une commande de moteur destinée à commander et/ou réguler le moteur électrique, procédé selon lequel on subdivise une plage globale de vitesse de rotation de la machine chirurgicale en au moins une plage de vitesse de rotation inférieure pour les faibles vitesses de rotation et au moins une plage de vitesse de rotation supérieure pour des vitesses de rotation plus élevées que celles de ladite au moins une plage de vitesse de rotation inférieure, et selon lequel on exécute dans ladite au moins une plage de vitesse de rotation inférieure, un premier procédé de commande et/ou de régulation pour commander et/ou réguler le moteur électrique, **caractérisé en ce que** dans ladite au moins une plage de vitesse de rotation supérieure on exécute un deuxième procédé de commande et/ou de régulation pour commander et/ou réguler le moteur électrique, et **en ce que** le premier procédé de commande et/ou de régulation et un procédé de modulation de largeur d'impulsion selon le principe du vecteur spatial (procédé SVPWM), pour lequel tous les enroulements de moteur sont alimentés en courant simultanément.

14. Procédé selon la revendication 13, **caractérisé en ce que** le premier et/ou le deuxième procédé de commande et/ou de régulation sont des procédés de modulation de largeur d'impulsion (PWM).

15. Procédé selon la revendication 13 ou la revendication 14, **caractérisé en ce que** l'on utilise un moteur électrique, qui est un moteur à courant continu sans balais.

16. Procédé selon l'une des revendications 13 à 15, **caractérisé en ce que** l'on utilise un moteur électrique, qui est un moteur électrique sans détecteurs.

17. Procédé selon l'une des revendications 13 à 16, **caractérisé en ce que** le moteur comporte des détecteurs de relevé de vitesse de rotation, et **en ce que** le premier procédé de commande et/ou de régulation est un procédé pour commander et/ou réguler la machine chirurgicale pour lequel la commande de moteur fournit des signaux de commande pour le moteur électrique en fonction d'une vitesse de rotation instantanée déterminée par les détecteurs de relevé de vitesse de rotation.

18. Procédé selon la revendication 17, **caractérisé en ce qu'**il est prévu un système de Hall pour relever une vitesse de rotation instantanée du moteur électrique, et **en ce que** le système de Hall comprend les détecteurs de relevé de vitesse de rotation.

19. Procédé selon l'une des revendications 13 à 18, **caractérisé en ce qu'**une valeur limite de vitesse de rotation entre ladite au moins une plage de vitesse de rotation inférieure et ladite au moins une plage de vitesse de rotation supérieure, reste inchangée pendant le fonctionnement de la machine.

20. Procédé selon l'une des revendications 13 à 18, **caractérisé en ce que** l'on modifie une valeur limite de vitesse de rotation entre ladite au moins une plage de vitesse de rotation inférieure et ladite au moins une plage de vitesse de rotation supérieure, pendant le fonctionnement de la machine.

21. Procédé selon l'une des revendications 13 à 20, **caractérisé en ce que** qu'une commutation du premier procédé de commande et/ou de régulation au deuxième procédé de commande et/ou de régulation s'effectue pour une première vitesse de rotation de commutation, et **en ce qu'**une commutation du deuxième procédé de commande et/ou de régulation au premier procédé de commande et/ou de régulation s'effectue pour une deuxième vitesse de rotation de commutation.

22. Procédé selon la revendication 21, **caractérisé en ce que** la première vitesse de rotation de commutation est égale ou supérieure à la deuxième vitesse de rotation de commutation.

23. Procédé selon l'une des revendications 13 à 22, **caractérisé en ce que** la commutation du premier procédé de commande et/ou de régulation au deuxième procédé de commande et/ou de régulation s'effectue automatiquement lors du passage de ladite au moins une plage de vitesse de rotation inférieure à ladite au moins une plage de vitesse de rotation supérieure et inversement.

24. Procédé selon l'une des revendications 13 à 23, **caractérisé en ce que** l'on utilise un moteur électrique à trois enroulements de moteur.
